# EUROPEAN PATENT SPECIFICATION

(11) **EP 0 469 745 B1**
(45) Date of publication and mention of the grant of the patent: **03.12.1997**
(21) Application number: 91306457.2
(22) Date of filing: 16.07.1991
(51) Int. Cl.: A61L 15/16, A61K 9/70, A61K 47/36

(54) **Transdermal medicine delivery apparatus**
Vorrichtung zur transdermalen Arzneimittelabgabe
Dispositif pour la délivrance transdermique de médicaments

(30) Priority: 21.07.1990 GB 9016056
(43) Date of publication of application: 05.02.1992
(73) Proprietor: CHATFIELD PHARMACEUTICALS LIMITED, London SW5 9JL (GB)
(72) Inventor: Solomon, Montague Cecil, London SW3 4QG (GB); Oraceska, Biljana, London SW10 (GB)
(74) Representative: SERJEANTS

(56) References cited:
- EP-A- 0 099 758
- EP-A- 0 289 342
- EP-A- 0 307 187
- US-A- 3 962 414
- US-A- 3 996 934
- DATABASE WPI Section Ch, Week 8002, Derwent Publications Ltd., London, GB; Class A, AN 80-02671C; & JP-A-54 151 115

## Description

### Technical Field

The invention relates to devices whereby medicaments can be administered through intact skin to the human or animal body. The medicine is absorbed into the blood circulation and transported to target tissue. By avoiding first pass, bioavailability is increased, therapeutic efficacy can be optimized, and side effects reduced.

### Background Art

A medicated plaster may comprise a backing material coated with an adhesive, and a reservoir of medicine adhering to a central part of the backing. The backing material should be impermeable to the medicine. When the periphery of the backing adheres to the skin, the reservoir of medicine is contacted with the skin, and the medicine permeates the skin.

The reservoir may comprise a hydrophilic or lipophilic polymer matrix in which the medicine is dispersed. The medicated polymer can be moulded into a disc of determined surface area and thickness. The release of the medicine is governed by its diffusivity in the matrix and absorbability by the skin. The problem is to design a therapeutic system in which the skin penetration rate is determined by the rate at which the medicine is delivered to the skin surface and not the inherent permeability of the skin. See Patent Specifications US 3962414 and 4031894, and EP 33615 A2.

### The Invention

A device according to the invention comprises a reservoir matrix having a medicine dispersed therein, and a membrane for controlling the rate of release of the medicine from the matrix, the matrix or the membrane comprising a cross-linked alginate polymer. The device may include means for keeping the membrane in contact with the skin, such as an adhesive plaster or bandage.

The cross-linked alginate polymer is biodegradable, and should be selected having regard to its chemical nad physical reactivity with the medicine. Molecular and structural factors such as polarity, hydrogen bonding, glass transition temperature, and the diffusivity of the medicine in the polymer are important. The alginic acid molecule is a long chain of uronic acid units joined by 1,4-glycosidic linkages. Thus it is a polyuronic acid made up of D-mannuronic and L-guluronic acid residues. The proportion of mannuronic and guluronic acid residues (the M/G) ratio) determines the release behaviour of the polymer with respect to the medicine: a faster release after swelling was found with a higher M/G ratio. Alginic acid salts with alkali metals, ammonia, magnesium and many organic bases are soluble in water, while those with most di- and polyvalent metals are insoluble. Calcium is most widely used for changing the viscosity and gel characteristics of algin solutions.

The medicine dispersed in the polymer matrix may for example be nicotine, which has high skin permeability and is used medically in this way to reduce the effects of nicotine withdrawal on cessation of smoking, diltiazem, verapamil, nifedipine or prazosin. The medicine can be incorporated in the polymer matrix by mixing a solution of the medicine with the polymer. The polymer may be partly cross-linked, for example by the introduction of calcium, copper, chromium or like divalent metal, ions to form the three-dimensional polymer matrix. The resulting matrix can be cast or spread on the backing material and allowed to dry. The cross-linking can be completed by spraying a di- or polyvalent salt solution on the dry polymer matrix. A 5% aqueous solution of calcium, copper or chromium ions is suitable for this purpose.

The membrane for controlling the rate of release of the medicine from the polymer matrix may be a polymer similar to that in the matrix. The polymer control release membrane can be cross-linked as described above to form an insoluble film.

The polymers are natural, inexpensive, non-toxic and bio-degradable. They may be used as the carrier matrix, or as the control release membrane, or both in a transdermal delivery system. The release rate from the transdermal delivery system can be controlled by the polymer concentration, drug concentration, control release membrane thickness, and cross-linking with metal ions.

### Drawings

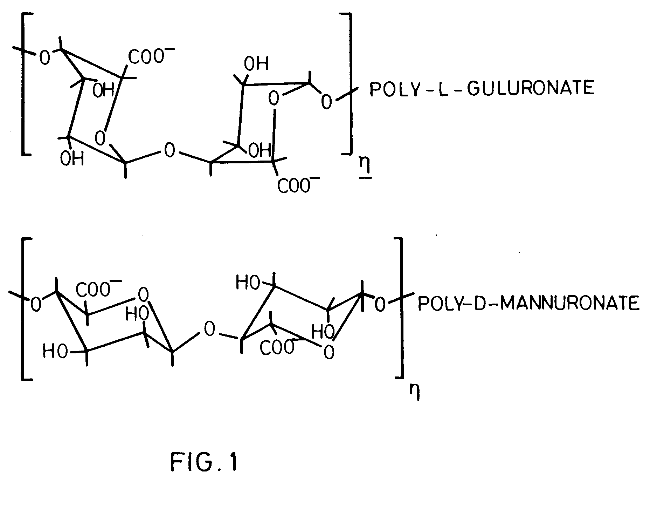

Figure 1 shows the structural formulae of poly-L-gulronate and poly-D-mannuric blocks comprising mixed sequences of which make up alginate polymers; and

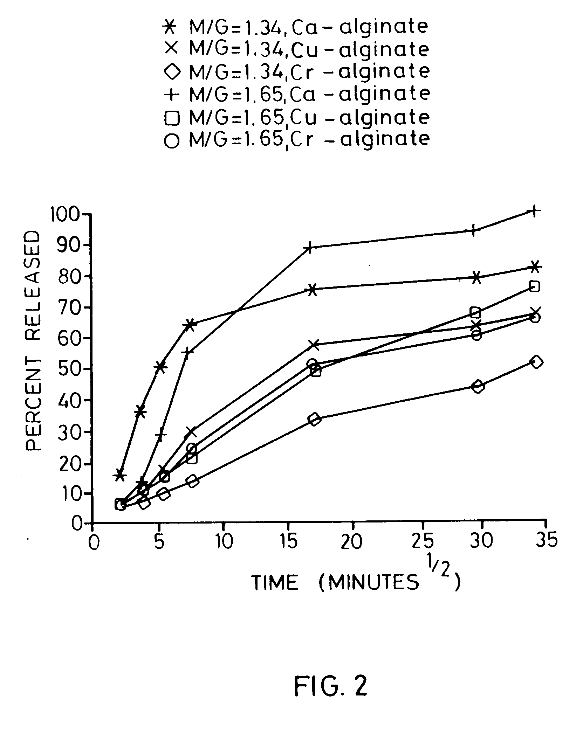

Figure 2 is a graph showing the percentages of nicotine released against time from a number of different alginate polymer matrices at 37°C as determined in a Franz-type diffusion cell.

### Example

### Polymer Matrix

7 g sodium alginate was homogenized with 3.5 g glycerol for 5 minutes. 0.5 g nicotine base was dissolved in 89 g distilled water. The homogenized alginate was mixed with the nicotine solution. The resulting viscous solution was cast using a thin layer chromatography spreader over a backing material and left overnight to dry. A 5% aqueous solution of calcium ions was sprayed over the dry alginate film, and left overnight to allow cross-linking to be completed. The resulting cross-linked film was cut into 3 cm diameter circles and stored in a desiccator.

### Control Release Membrane

3 g sodium alginate was homogenized with 3.5 g glycerol for 5 minutes and mixed with 89.5 g distilled water in the absence of any medicine. The resultant viscous solution was cast using a thin layer chromatography spreader over a backing material and left overnight to dry. A 5% aqueous solution of calcium ions was sprayed on the dry film. The film was left overnight to allow cross-linking to be completed. The cross-linked film was cut into 3 cm diameter circles and stored in a desiccator.

A device for transdermal delivery of the nicotine through intact skin was assembled by placing a circle of the polymer matrix and a circle of the control release membrane in sequence on a piece of backing material so that the matrix was held in position by the control release membrane. The device could then be applied to the skin of a patient by contact through the membrane, which was held in place by an adhesive contacting the surrounding skin.

Release of the medicine comprises an initial burst as a result of hydration of the polymer matrix particularly when the amount of medicine in the polymer matrix was large. This was followed by steady state permeation with square root release kinetics. The initial burst is believed to be due to medicine present on the surface of the matrix resulting from the drying of the solution in the preparation process. In the steady state permeation, the medicine diffuses through pores in the matrix which is much slower. When the control release membrane is added to the device, the initial burst is suppressed due to the control release properties of the membrane, and this release follows with square root of time release kinetics.

## Claims

1. A transdermal medicine delivery device which comprises a reservoir matrix having a medicine dispersed therein characterized by a membrane for controlling the rate of release of the medicine from the matrix, the matrix or the membrane comprising a cross-linked alginate polymer.

2. A transdermal medicine delivery device according to claim 1 in which the matrix is cross-linked.

3. A transdermal medicine delivery device according to claim 1 or claim 2 in which the membrane is cross-linked.

4. A device according to any preceding claim which includes backing material.

5. A device according to any preceding claim in which the polymer is cross-linked by the introduction of calcium ions.

6. A device according to any preceding claim in which the medicine is selected from the group consisting of nicotine, diltiazem, verapamil, nifedipine or prazosin.

## Patentansprüche

1. Einrichtung zur transdermalen Arzneimittelabgabe, die eine Reservoir-Matrix aufweist, in der ein Medikament dispergiert ist, **gekennzeichnet** durch eine Membran zum Steuern der Geschwindigkeit der Abgabe des Medikaments von der Matrix, wobei die Matrix oder die Membran ein vernetztes Alginat-Polymer aufweisen.

2. Einrichtung zur transdermalen Arzneimittelabgabe nach Anspruch 1, wobei die Matrix vernetzt ist.

3. Einrichtung zur transdermalen Arzneimittelabgabe nach Anspruch 1 oder Anspruch 2, wobei die Membran vernetzt ist.

4. Einrichtung nach einem der vorhergehenden Ansprüche, wobei sie ein Trägermaterial aufweist.

5. Einrichtung nach einem der vorhergehenden Ansprüche, wobei das Polymer durch die Einfügung von Kalziumionen vernetzt ist.

6. Einrichtung nach einem der vorhergehenden Ansprüche, wobei das Medikament aus der Gruppe ausgewählt ist, die aus Nikotin, Diltiazem, Verapamil, Nifedipin oder Prazosin besteht.

## Revendications

1. Dispositif de délivrance transdermique de médicament, qui comprend une matrice réservoir dans laquelle est dispersé un médicament, caractérisé par une membrane pour régler la vitesse de libération du médicament hors de la matrice, la matrice ou la membrane comprenant un polymère d'alginate réticulé.

2. Dispositif de délivrance transdermique de médicament selon la revendication 1, dans lequel la matrice est réticulée.

3. Dispositif de délivrance transdermique de médicament selon la revendication 1 ou la revendication 2, dans lequel la membrane est réticulée.

4. Dispositif selon l'une des revendications précédentes, qui comprend un matériau support.

5. Dispositif selon l'une des revendications précédentes, dans lequel le polymère est réticulé par introduction d'ions calcium.

6. Dispositif selon l'une des revendications précédentes, dans lequel le médicament est choisi dans le groupe constitué par : nicotine, diltiazem, vérapamil, nifédipine ou prazosine.
